# EUROPEAN PATENT APPLICATION

(11) **EP 4 354 136 A1**
(43) Date of publication of application: **17.04.2024**
(21) Application number: 22201755.0
(22) Date of filing: 14.10.2022
(51) Int. Cl.: G01N 33/03

(54) **A METHOD FOR EVALUATING THE AUTHENTICITY OF FOOD PRODUCTS AND A SYSTEM THEREOF**

(71) Applicant: National and Kapodistrian University of Athens (NKUA), Special Account for Research Grants (SARG), 105 61 Athens (GR)
(72) Inventor: THOMAIDIS, Nikolaos, 145 61 Athens (GR); AALIZADEH, Reza, 11526 Athens (GR); KRITIKOU, Anastasia, 19100 Megara Attica (GR); DRAKOPOULOU, Sofia, 15722 Athens (GR)
(74) Representative: Minas, Nikolaos

(57) **Abstract**

The present disclosure relates method and a system for evaluating the authenticity of food products, by processing lipid content information of a food product sample extracted from at least one Total Ion Spectrum, TIS, over a predetermined region of interest, ROI. The extracted lipid content information is processed by means of a trained neural network to evaluate the authenticity of the food product sample and accordingly generate an authentication score indicative of the correlation between the analysed food product sample and one or more food product classes.

## Description

### Field

The present invention relates to a methodology and a system for analysing food samples to determine product authenticity and ensure quality of the product and its compliance with particular specifications. The present invention further relates to a food traceability system that is configured to trace and authenticate food products at different steps of the supply chain.

### Background

During the last two decades, food authenticity studies have been found on the frontline of scientific research. The development in the field of food science aimed to achieve high nutritious, superior quality and safe food. Global recognition of products, known for their unique nutritional properties, such as olive oil and dairy products, has raised awareness for quality assurance. The financial importance of these foods leads producers to various fraudulent practices, such as mislabelling or adulteration of products with illegal substances for profit purposes.

According to the established legislative frameworks, targeted activities are being made by the European Union, encouraging scientific parts for the development of technologies and methods to detect fraud and ensure products' authenticity [Regulation (EC) No 178/2002 General Food Law)]. Under the term of authenticity, we primarily refer to the quality assurance of the food product and its compliance with the particular specifications, as stated on the label. Special attention is also be paid to food origin verification (e.g., geographical/animal origin and variety) that may refer to a place or region in which the food or its ingredients produced, the botanical species and/or raw material used etc. Therefore, in order to protect food produced in certain member states or regions according to specific conditions, the European Economic Community has established a specific legal framework by introducing the terms "Protected Designation of Origin" (PDO) and "Protected Geographical Indication" (PGI), established by the Council Regulation (EC) No 2081/92 and No 2082/92.

Moreover, under the horizontal European "Farm to Fork strategy", integrated food traceability systems are strongly suggested and imposed for food industries. Under EU law, "traceability" means the ability to trace any food, feed, food-producing animal or substance that will be used for consumption, through all stages of production, processing and distribution. Traceability is a way of responding to potential risks that can arise in food and feed, to ensure that the product not only meets the quality standards but foremost it is safe for consumption by EU citizens. It is vital for both national authorities and food industries to identify potential risk and through the traceability system be able to isolate the problem and prevent contaminated products from reaching consumers.

Therefore, there is an imperative need to provide solution for tracing the authenticity of food products at different stages of the supply chain, from production to consumption.

### Summary

The aim of the present invention is to provide a methodology and a system for authenticating food matrices of raw materials and/or food products.

A further aim of the present invention is to provide a system and a method for tracing food products along the supply chain.

The above aims are being achieved with the method and system presented in the independent claims, while preferred embodiments are described in the dependent claims.

According to embodiments of the present invention, a method is provided for evaluating the authenticity of food products, the method comprising;
receiving at least one Total Ion Spectrum, TIS, representing a chemical profile of a corresponding food product obtained from a full scan mass spectral analysis of a corresponding food product sample; and
evaluating by mean of a trained neural network the authenticity of the food product sample by performing the steps of:
   processing the at least one TIS to extract chemical characteristics of the analysed food product sample over at least one Region of Interest, ROI, each ROI associated with a chemical signature of one or more food product classes over a predetermined region of the TIS,
   correlating the extracted chemical characteristics with the corresponding chemical signature of each food product class over the at least one ROI; and
   outputting, based on the correlation, an authentication score indicative of the correspondence between the food product sample and each of the food product classes.

According to an embodiment, the step of processing the at least one TIS comprises the steps of;
converting each TIS into an image;
processing image pixels associated with each ROI to extract corresponding pixel intensity values and
determining, based on the extracted pixel intensity values, the chemical characteristics of the analysed food product over the predetermined ROI

According to an embodiment, the Total Ion Spectrum represents an averaging of the values extracted from a plurality of full scan mass spectrums obtained during the spectral analysis.

According to an embodiment, the Total Ion Spectrum represents the lipid profile of the analysed food product sample and the extracted chemical characteristics represent the extracted lipid content, ELC, of the food product sample at each ROI

According to an embodiment, the chemical characteristics extracted from the one or more ROIs of the TIS comprise lipid content information of the food product sample obtained from detected peak intensity values and corresponding mass-to-change ratio based on the extracted intensity values of the corresponding pixels.

According to an embodiment, the trained neural network is a convolution neural network comprising at least two convolutional layers, at least two fully connected layers, and a plurality of max-pooling and dropout layers.

According to an embodiment, the number of convolution layers is adjusted depending on the number of food product classes to be associated with the analysed food product matrix.

According to an embodiment, the food authenticity score is indicative of an adulteration status and/or an authenticity status of the analysed food sample.

According to an embodiment, the authentication score is represented as a probability indicative of the correlation between the food product sample and the one or more food product classes.

According to an embodiment, the method further comprises the step of generating a scannable QR code comprising information associated with any one, or a combination of: the authentication score, a detected food product class, chemical composition information and nutritional information obtained from the spectral analysis, and adulteration status.

According to an embodiment, the method further comprises the step of communicating the QR code to a user via a user interface of a software application running on an electronic device.

According to an embodiment, the method further comprises the step of at storing authentication information associated with the analysed food product sample in a distributed ledger technology, DLT, running on a cloud computer server, the authentication information comprising any one, or combination, of ; authentication score, food product class associated with the analysed food product sample, an origin information, chemical composition, nutritional information, and location where the food product sample was obtained.

According to an embodiment, the TIS is generated from a plurality of full scan mass spectrums obtained from a MALDI-TOF-MS spectral analysis of the food product sample.

According to a second aspect of the present invention, a digital system is provided for evaluating the authenticity of a food product sample, the system comprising a
a user interface running on an electronic device accessible by a user for receiving and providing information;
processing module configured to execute the method of the embodiments of the first aspect for evaluating the authenticity of a food product sample; and
a communication module configured to output authentication information associated with the analysed food product sample.

According to a further aspect a computer program product comprising executable instructions, which when executed causes a processing unit to perform the method of embodiments of the first aspect.

### Brief Description of the drawings

The following drawings are provided as an example to explain further and describe various aspects of the invention.
Figure 1 shows an exemplified implementation of the workflow for analysing food samples to determine their authenticity according to embodiments of the present invention.
Figure 2 shows an exemplified implementation a sample analysis stage of the system shown in Figure 1 according to embodiments of the present invention.
Figure 3 shows an exemplified method for performing sample analysis in the sample analysis stage according to embodiments of the present invention.
Figure 4 shows an example of a Total Ion Spectrum (TIS) representing the lipid profile of the analysed food product sample obtained from an instrumental analysis of a corresponding food product sample according to embodiments of the present invention.
Figure 5 shows an exemplified implementation of a data processing stage of the food authenticity evaluation of the system presented in Figure 1 according to embodiments of the present invention.
Figure 6 shows an exemplified architecture of a convolution neural network used in the data processing stage according to embodiments of the present invention.
Figure 6a shows exemplified Total Ion Spectrums (TIS) obtained from Extra Virgin Olive Oil (EVOO) and Refined Olive Oil (ROO) respectively and their triacylglycerol (TAG) profile associated with their lipid content over the corresponding Regions of Interests (ROIs) according to embodiments of the present invention.
Figure 7 shows an exemplified implementation of the system of the Figure 1 according to embodiments of the present invention.
Figure 8 shows an exemplified user interface displaying the information obtained from the analysis of the food product sample.
Figure 9 shows an exemplified method for determining the authenticity of a food product sample according to embodiments of the present invention.

### Detailed Description

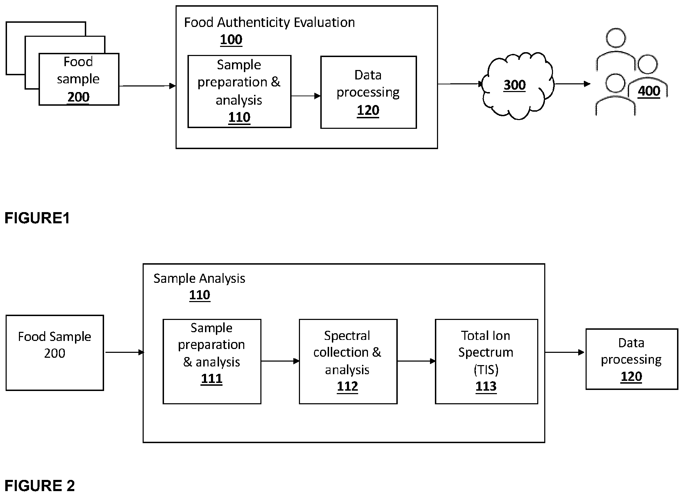

The functionality of the proposed system and method will be illustrated using the exemplified implementations shown in the figures 1 to 9, which will be described in more detail below. It should be noted that any references made to specific types of data are only indicative and do not restrict the proposed functionality in any way. While the proposed system and method have been shown and described with reference to certain illustrated embodiments thereof, it will be understood by those skilled in the art that various changes in form and details may be made therein without departing the proposed functionality.

In general, the present invention relates to the development of a Machine Learning model to investigate the authenticity and detect potential adulteration of foods of plant and animal origin. Data introduced in the ML model are derived by MALDI-TOFMS analysis, exploiting the lipid profile of food of interest e.g. olive oil, milk, and the like. The ML model predicts the origin of each food of interest according to feedback parameters associated with the corresponding food class (e.g., determining the specific lipid class which is important for the studied food product). For instance, some food products have distinguished product-origin lipid content. Such information is used in ML to trace authentic products from different animals. For example, dairy products such as milk, have a distinguished animal-origin lipid content e.g. goat, cow, and the like. The feedback parameters are adjusted and optimized based on the training of the neural network. Specifically, the food authenticity is investigated based on the convolutional neural network (CNN), which uses the lipid profile of food, as obtained from MALDI-TOFMS analysis. The algorithm then generates a Region of Interest (ROI) for each extracted lipid spectrum and correlates these ROIs with the origin of their food in a supervised technique.

Figure 1 shows an exemplified implementation of a process for evaluating the authenticity of food product according to embodiments of the present invention. The exemplified process comprises receiving food product samples 200, which are processed at a food authenticity evaluation stage 100 to determine their authenticity and detect adulteration of the food product. The authenticity information obtained from the food authenticity evaluation stage 100 may be stored in a desired format and may further be access by and/or communicated to users via a predetermined interface e.g. software application, website interface, and the like. The food authenticity evaluation step comprises a sample analysis phase 110 and a data processing phase 120. At the sample analysis stage 110 the food samples are processed and analysed, while at the data processing phase 120 the data obtained from the food sample analysis during phase 110, is processed to extract chemical characteristics of the analysed food products that are used to determine their authenticity and generate a corresponding authentication score. The authentication score may be in the form of a probability value and may be communicated to an authenticity cloud platform 300 accessed by authorised users 400 such as food producers, consumers, food authorities, and the like.

Figure 2 shows an exemplified implementation of the sample analysis phase 110 according to embodiments of the present invention. The sample analysis phase 110 may comprise a sample preparation step 111, whereby the food samples are prepared before they are analysed during a spectral analysis step 112. During the spectral analysis step 112, the prepared food samples are scanned using a spectral analysis instrument. The output of step 112 is one or more full scan mass spectrum, which are combined into a Total Ion Spectrum (TIS) 113a, which is then fed to the data processing phase 120. The TIS 113a represents the chemical profile of the analysed food product sample over a specified mass to charge ratio, m/z. the TIS (total ion spectrum) is a spectrum created by summing up intensities of all mass spectral peaks belonging to the same scan. In the present invention, the TIS would be exemplified as representing the lipid profile of the analysed food product sample (e.g. olive oil, milk).

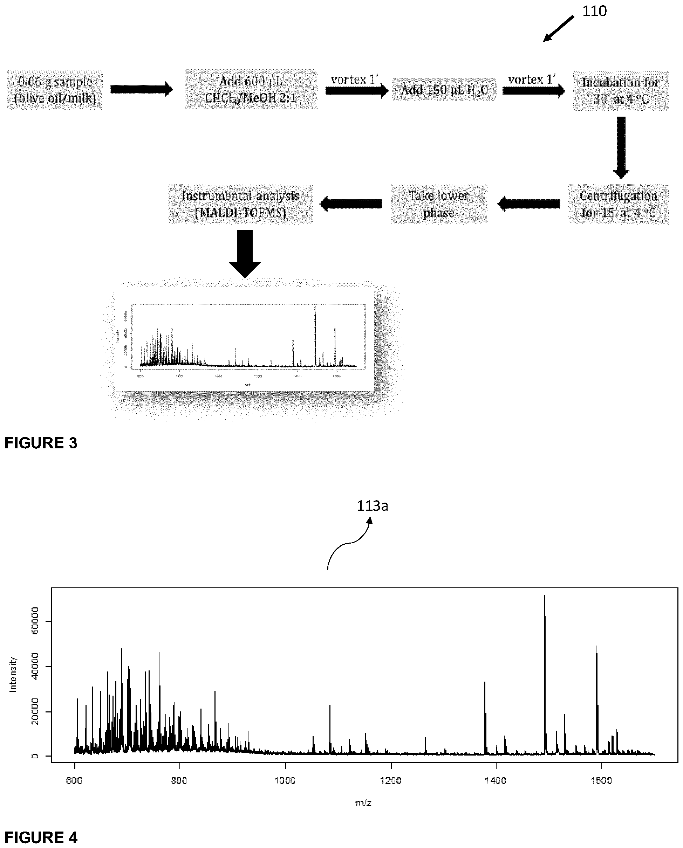

An exemplified food sample preparation protocol performed during the sample analysis phase 110 is shown in figure 3. In brief during the sample preparation step 111, 0.06 g of all samples were extracted with 0.6 mL of solution containing chloroform (CHCl₃): methanol (MeOH) (1:2) (v/v) and then vortexed for 1 min. Subsequently, 0.15 mL of distilled water (H₂O) were added to the samples and vortexed again for 1 min. Samples were incubated at 4° C for 30 min. Following incubation, samples were centrifuged at 10000 rpm at 4° C, upper layer was aspirated to waste and lower organic phase was collected. Part of the samples included in the study were prepared (extracted) in triplicates to verify the repeatability of the procedure. MALDI-TOFMS, or an equivalent instrumental analysis method (e.g., other High Resolution Mass Spectrometry (HRMS) -based techniques, such as LC-HRMS, GC-HRMS) is performed at step 112 to analyse the prepared food sample. For the analysis, α-Cyano-4-hydroxycinnamic acid (HCCA) was used as MALDI food matrix. For the MALDI target preparation, a ground steel MALDI target plate may be utilised and a "double layer" method may be carried out for the spotting of the extracted samples. Specifically, individual target positions on the MALDI plate may be covered with a thin layer of a saturated HCCA solution, prepared in CHCl₃: MeOH 1/2 v/v. Following HCCA deposition, the target plate is allowed to dry at room temperature. Sample's extracts may be mixed in 1:1 ratio with the previous saturated HCCA solution. 0.5 µL of the resulting mixtures applied to the previously prepared positions on the target plate. The target plate may be again allowed to dry at room temperature before MALDI-TOFMS analysis. During the MALDI-TOFMS analysis at step 112, lipid profiles from all samples are collected using, for example, a benchtop microflex LRF model MALDI-TOF mass spectrometer equipped with a nitrogen laser at 337 nm and using a repetition rate of 60 Hz. MALDI-TOFMS may be operated in reflectron detection mode with positive ion acquisition between 0.6 and 1.7 kDa m/z range. Mass spectra automatically collected, summing 1200 shots for each sample. Random walk mode (partial spot) used, with 50 shots collected at each raster position. Regarding the laser adjustment, the laser power adjusted across a narrow (relative power) range to avoid detector saturation. "Peptide calibration standard was used for external quadratic calibration. The output of the MALDI-TOFMS analysis is a full scan mass spectrum representing the chemical profile of the analysed food product. In embodiments of the present invention, the TIS may represent a lipid profile of the analysed food product sample and can be applied to different food product matrices that have a lipid background such as oil, fish, meat, dairy, and similar type products. An exemplified TIS representing the lipid profile of a food product is shown in figure 4. In the case of olive oil and dairy products, figure 4 represents the characteristic mass spectrum of the significant lipid classes found in these food matrices. The TIS may be further optimised to represent different aspects of the food product sample such as the metabolic profile by adjusting the boundaries of the region of interest, ROI, e.g. by changing the m/z range of the analysis representing the boundaries of the corresponding ROI.

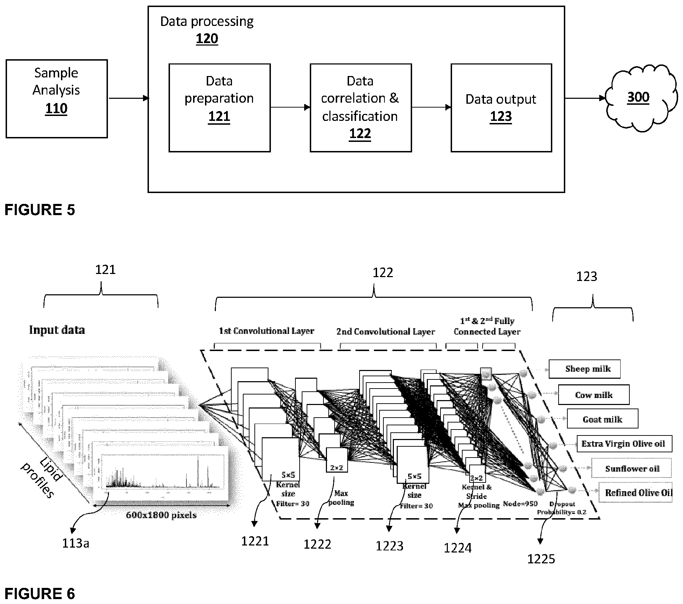

Figure 5 shows an exemplified implementation of the data processing stage 120 in food authenticity evaluation phase 100 according to embodiments of the present invention. The data processing stage is provided with a data preparation step 121 whereby each of the TISs 113a generated as output from the sample analysis stage 110 is converted into an image. The image is preferably a grey scale image having a predetermined resolution e.g. 600X1800 pixels. However, it should be noted, that the image could also be an RGB image or a black & white image. By converting the data associated with each TIS 113a into an image, it becomes easier to extract relevant information from the total ion Spectrum (TIS) that is unique for a class of food products. The generated TIS includes information such as detected m/z and their relative intensities, and/or retention time. The generated TIS image is used by a trained neural network at step 122 to extract relevant chemical characteristics of the Spectrum such as the lipid content of the food product sample over one or more Region of Interest (ROI). During the correlation and classification step 122, the neural network extracts chemical characteristics from the received TISs over one or more ROIs, which are then classified and correlated with chemical characteristics of one or more food product classes. In response to the correlation, an authentication score is generated at step 123 indicating the correspondence between the extracted chemical characteristics the food product sample and the chemical characteristics associated with one or more of the food products classes. For example, the authentication score may be provided in the form of a probability value indicating the how closely the chemical characteristics of the analysed food product sample resemble the expected chemical characteristics of one or more food product classes. Based on the assigned authentication score, it becomes easier to determine the authenticity of the analysed food product sample and detect adulteration attempts. For example, when testing a sample taken from olive oil marketed as extra virgin, it is expected that the authentication score generated would indicate a high probability value for the extra virgin olive oil food product class. As such, if the resulting probability is lower than a predetermined threshold it would indicate an adulteration attempt in the analysed olive oil sample. A QR code may be generated at the data output step 123 comprising information extracted during the data processing stage of each TIS. The information encoded in the QR code may be anyone, or a combination, of the generated authentication score, the representative TIS image, chemical composition, nutritional information, origin, and the like. The generated QR code may be applied to the food product packaging to enable retrieval of the information via an electronic device, e.g. via scanning of the QR code. The data processing stage 120 is running on a computer hardware device, and may be accessed by a user through a user interface to interact with the different stages of the data processing stage 120.

For example, food companies can obtain an encrypted QR code which includes adulteration status, chemical analysis and authenticity results of their food product and put this searchable QR code in their food product for consumers. Then, the consumers can trace how the product is made using their mobile phone to scan the QR code and extract product information such as, what chemicals exist in the product, nutritional values, adulteration level and corresponding authenticity status from different stages of the supply chain e.g., from cultivation to packaging and delivery. The information collected during the data processing stage 120 may be stored in a distributed ledger technology (DLT) system such as a blockchain, which may be used to verify the authenticity of the food product in the market.

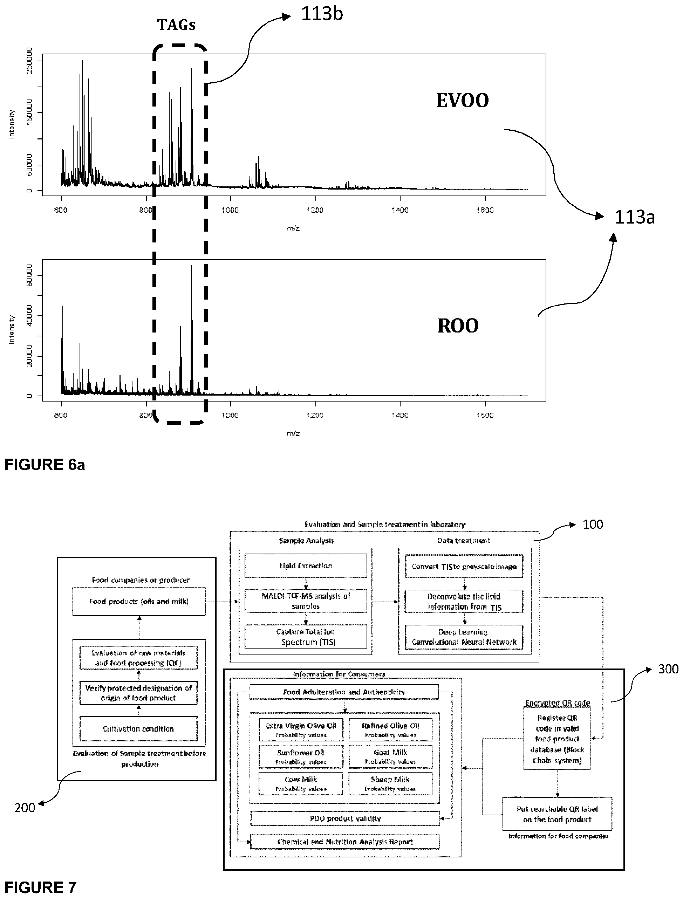

Figure 6 shows an exemplified architecture of a convolution neural network (CNN) model configured to run on a computer hardware component and used for evaluating the authenticity of food product samples according to embodiments of the present invention. The CNN model was created based on Deep learning conventional neural network (DL-CNN), trained on TIS images of different food class products such as milk and oil-based products e.g. goat milk, sheep milk, cow milk, extra virgin olive oil, sunflower oil, refined oil, and the like. The trained CNN is used during the data processing stage 120 to process and classify/correlate chemical characteristics of a food product sample extracted to a selected set of food product classes and accordingly determine the authenticity of the food product sample. The use of a CNN for processes TIC images greatly simplifies the authentication process since it does not require the use of complex peak picking/detection techniques. Peak picking procedure often requires optimization of peak detection algorithm. For instance, CentWave algorithm or Gaussian fitting functions in case of profile mode peaks requires optimization of peak width, signal to noise ratio, local peaks shift due to matrix, and mass accuracy fluctuations. These parameters are not global, and they need to be optimized after evaluating the individual samples and find thresholds which can trade-off between correct peak detection and reasonable peak parameters. DL-CNN does not require such optimization and instead learn directly from samples in relation to their classes. This way, if a peak shifts due to matrix of the food product sample will be included in region of interest of the model in case of specific food product. As shown in figure 6, the proposed DL-CNN model used in the data processing stage 122 may be provided with two convolutional layers, two fully connected layers, 950 hidden layers and six nodes, as well as max-pooling (using rectifier function) and dropout layers with probability of 0.2. The exemplified DL-CNN was created using a "mxnet" R package. The size of convolution window (Kernel), output channels (filter), strides of convolution, type of activation function, nodes and hidden layers were optimized using k-fold cross validation method. The exemplified CNN may be a ResNet-50, ResNet-101, ResNet-152, or similar. According to embodiments of the present invention, the 1^{st} and 2^{nd} convolutional layers extract relevant information from the total ion spectrum (TIS) that is unique for each preselected food product class. As such the proposed CNN model requires a TIS image as input generated during data preparation step 121 or directly from the instrumental analysis apparatus. Each TIS may be a 600×1800 pixels image representing the lipid profile of a product sample analysed using MALDI-TOF-MS. To determine the authenticity score for a food product sample, input data is fed into the CNN in the form of a greyscale TIS image having a predetermined resolution, although it should be noted that a colour or black & white TIS image may be equally used. The CNN processes the TIS image by performing successive convolution and pooling iterations 1221, 1222, 1223, 1224, to extract the intensity value of pixels in one or more Regions of Interest (ROIs) in the TIS image. The extracted pixel intensity values are directly linked to the specific lipid content information of the analysed food product sample. In essence, the pixel intensity value provides a digital chemical signature/fingerprint of the lipid content of the food product sample based on the intensity values of the detected peaks in the Region of Interests of the TIS. The digital signature/fingerprint may then be correlates with the expected digital signature of a representative food product class. For example, the CNN may check the intensity of pixels only at predetermined regions of interest (ROIs) in the TIS image associated with a representative food product class. Figure 6a shows an example of TISs 113a generated from Extra Virgin Olive Oil (EVOO) and Refined Olive Oil (ROO) and their corresponding triacylglycerol (TAG) profile, corresponding to lipid content information, over a predetermined Region of Interest (ROI) 113b selected based on the corresponding food product class. As it can be seen, the chemical signature of the lipid content over the ROI 113b is different for each product. Therefore, the intensity values of the corresponding pixels over the given ROI 113b would also be different, thus distinguishing the two food product samples as belonging to different, but related, food classes. As such, if a food product sample is labelled as EVOO but in actuality is Refined Olive Oil (ROO), the authentication score generated based on their lipid content profile over the corresponding ROI 113b, would classify the product as having a high probability being ROO compared to EVOO. The resulting intensity values are passed to the 1^{st} and second fully connected layers 1225, which is then correlated to the preselected food product classes. The final result of the analysis is the generation of an authentication score e.g. a floating-point number or a probability value, indicating the level of authenticity of the food product sample with respect to each of the tested food product classes. As such, the probability of a sample belonging to a food product class is in causation of its authenticity and adulteration level. For example, the closer the probability value is to 1.0, the lower the chances to an adulteration in the food product. The generated authentication score of the analysed food product sample may be encoded in a QR code. The generated QR code may be hosted in a secure food authenticity cloud platform 300 and shared with the users 400 such as producers, consumers, food authorities, and the like. The QR code may be applied to the food packaging and the information encoded may be accessed by the users via their mobile phone or another electronic device. The information encoded in the QR code may include in addition to the authentication score, information relates to adulteration status, detected food product class, nutritional information, chemical composition, and the like. Furthermore, the authentication score may be processed in authenticity cloud platform 300 to detect whether the food product sample meets certain authenticity criteria. If it is detected that the generated authentication score of a food product sample is lower than a predetermined threshold, the authentication cloud platform may raise a warning and/or an alert to a predetermined user such as a food authority, the producer, and the like.

The proposed CNN architecture shown in figure 6 was trained using =200 TIS lipid profiles taken from samples belonging to six classes of food product including "sheep milk" (n=40 samples), "cow milk" (n=42 samples), "goat milk" (n=40 samples), "extra virgin olive oil" (n=19 samples), "sunflower oil" (n=8 samples) and "refined olive oil" (n=11). A raster image having a predetermined lower resolution e.g. of 40×40 pixels may be created from each TIS and correlated to each class. DL-CNN was then used to learn from this knowledge-based classification and detect class of a suspect food product. The classification outcome is presented in probability values which can reveal information such as adulteration status or the authenticity of a sample.

Figure 7 shows a more detailed representation of the exemplified system for evaluating the authenticity of a food product shown in Figure 1. The food product samples 200 may be provided by food companies and/or producers at different stage of the supply chain. The food samples may have undergone an evaluation before the actual production of the product, which may generate a set of information associated with the cultivation conditions, the verification of the protected designation of origin, and evaluation of raw materials. The information may be passed as metadata along with the food product sample to the authenticity evaluation phase 100, which as described with reference to figure 1, it may comprise a sample analysis stage 110 and a data treatment stage 120. For example, and in accordance with embodiments of the present invention, during the sample analysis stage 120 the lipid profile of the food product is extracted from the food product sample, which is then analysed using a MALDI-TOFMS analysis to generate a corresponding Total Ion Spectrum, TIS. The TIS is passed to the data treatment stage 120, whereby it is converted to a grey scale image, which is then used to deconvolute the total lipid information over one or more Region of Interest (ROI) of the TIS associated with one or more food product classes. In general, the TIS includes information associated with many different lipid types. The deconvolution process tries to find/locate one or more segments of the TIC that are unique for each food matrix including its intensity value. The extracted lipid content (ELC) information obtained from each segment, also referred to as ROI, of the TISis then fed into a deep learning convolution neural network to determine the authentication score, as described previously. The authentication score is communicated to an authenticity evaluation platform, where the authentication score is encoded into a QR code along with other information associated with the food product sample such as information for consumers including the food adulteration and authenticity status, food product classification (extra virgin oil, refined oil, sunflower oil, goat milk, cow milk, sheep milk, etc), Protected Designation of Origin (PDO) of the product and its validity, and the chemical and nutritional analysis report. The QR code may be encrypted and stored in a valid food product database, which may be accessed by authorised users of the platform 300. The QR code may also be added to the packaging of the food product so that users can access the encoded information through the platform 300.

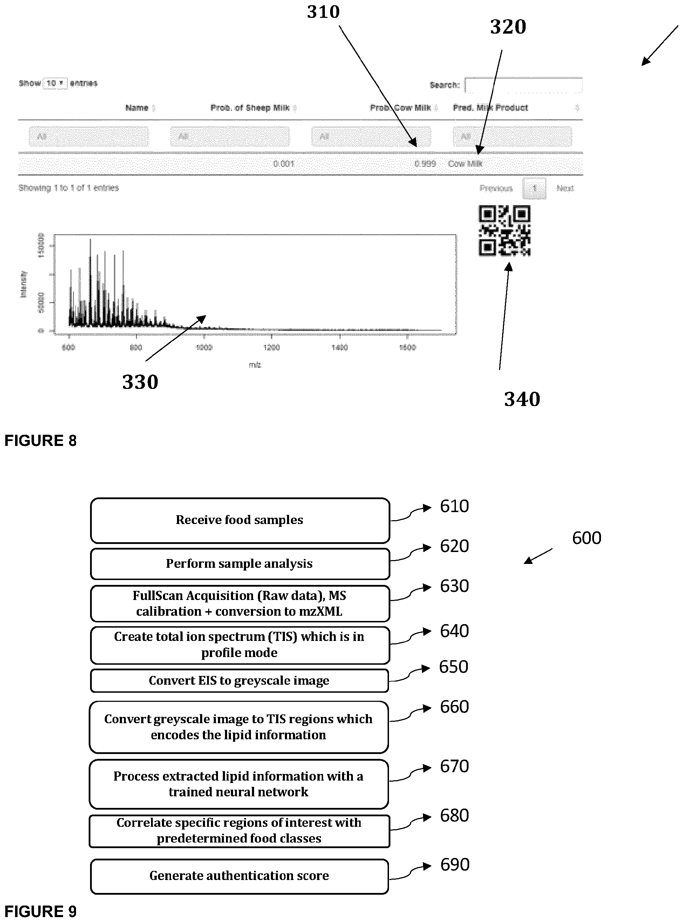

Figure 8 shows an example representation of a user interface of the cloud platform 300 according to embodiments of the present invention. As shown, a user may access via the QR code the encoded information stored in the cloud platform database. At a user interface running on an electronic device e.g. mobile phone, the encoded information in the QR code is displayed. For example, the information displayed may include, but not limited, to the corresponding Spectrum 330, the associated QR code 340, the authentication score assigned 310, and the food classification 320. Other information may also be displayed, as mentioned previously, such as nutritional and chemical analysis, POD validity, adulteration status, and the like.

Figure 9 shows an exemplified method 600 for evaluating the authenticity of a food product sample according to embodiments of the present invention. The method starts at step 610 where the food product samples 200 are received for authenticity evaluation. A sample preparation is performed at step 620, followed by a full mass spectrum scan at step 630 using MALDI-TOFMS or similar spectral analysis instruments, resulting in the generation of a representative total ion Spectrum (TIS) 113a of the lipid profile of the food product sample at step 640. The TIS is converted into a grayscale image at 650, which is subsequently processed at step 660 to determine region of interest (ROIs) which comprise lipid content information for one or more food product classes. At step 670, digital fingerprint information associated with the lipid content of the food product sample is extracted over the determined Region of Interests. The digital fingerprint information is extracted based on the intensity values of pixels in the one or more ROIs of the TIS image, which may be selected and/or determined based on food product class of the food sample e.g. TIS ROIs for olive oil are different from other oil based products The extracted TIS digital chemical signature/fingerprint information is processed by a trained neural network at step 680, whereby the extracted lipid content information is correlated with expected lipid content of predetermine food classes. The result of the correlation is an authentication score generated at step 690, which is indicative of the correspondence between the food product sample with one or more of the predetermined food product classes. The food product classes may be selected based on the training of the network and/or the food product sample to be evaluated. In general, the method of the present invention tries to identify sequence of pixels having an intensity value, also referred to as digital fingerprint information, that reveal the lipid content information of the food product sample based on peaks detected in the TIS and their corresponding intensity values. The extracted lipid content information may then be compared to expected lipid content information for a predetermined food product class. The resulting authentication score then reveals the matching relationship between the extracted and expected lipid content information over the one or more Regions of Interest.

The present invention provides a holistic approach towards food authenticity assessment and detection of possible adulteration, thereby enabling the certification of an authentication procedure regarding the food origin in various stages of the supply chain of the food product (processing, standardization, etc.). An authenticity digital platform 300 is further provided, which is accessible to the customers via an electronic device, thereby enabling for food products to be checked, certified in terms of authenticity and marked with QR-code, unique for each product. The QR code, may comprise information related to : (i) the authenticity assessment of the products via QR screening label on the standardized final product; (ii) traceability support process and documentation of authenticity at the stages of the production process regarding the origin of food (e.g., of animal origin). The authenticity digital platform may be running of a cloud server and be accessible by a plurality of users. The authenticity digital platform aggregates the information collected during the authenticity evaluation process, and also run some parts of the authenticity evaluation process such as the data treatment and processing stage 120.

## Claims

1. A method for evaluating the authenticity of food products, the method comprising;
receiving at least one Total Ion Spectrum, TIS, representing a chemical profile of a corresponding food product obtained from a full scan mass spectral analysis of a corresponding food product sample; and
evaluating by mean of a trained neural network the authenticity of the food product sample by performing the steps of:
processing the at least one TIS to extract chemical characteristics of the analysed food product sample over at least one Region of Interest, ROI, each ROI associated with a chemical signature of one or more food product classes over a predetermined region of the TIS,
correlating the extracted chemical characteristics with the corresponding chemical signature of each food product class over the at least one ROI; and
outputting, based on the correlation, an authentication score indicative of the correspondence between the food product sample and each of the food product classes.

2. The method of claim 1, wherein the step of processing the at least one TIS comprises the steps of;
converting each TIS into an image;
processing image pixels associated with each ROI to extract corresponding pixel intensity values and
determining, based on the extracted pixel intensity values, the chemical characteristics of the analysed food product over the predetermined ROI.

3. The method of claim 2, wherein the Total Ion Spectrum represents an averaging of the values extracted from a plurality of full scan mass spectrums obtained during the spectral analysis.

4. The method of claims 1 to3, wherein the Total Ion Spectrum represents the lipid profile of the analysed food product sample and the extracted chemical characteristics represent the extracted lipid content, ELC, of the food product sample at each ROI.

5. The method of claim 2 to 4 , wherein the chemical characteristics extracted from the one or more ROIs of the TIS comprise lipid content information of the food product sample obtained from detected peak intensity values and corresponding mass-to-change ratio based on the extracted intensity values of the corresponding pixels.

6. The method of any one of the preceding claims, wherein the trained neural network is a convolution neural network comprising at least two convolutional layers, at least two fully connected layers, and a plurality of max-pooling and dropout layers.

7. The method of any one of the preceding claims, wherein the number of convolution layers is adjusted depending on the number of food product classes to be associated with the analysed food product sample.

8. The method of any one of the preceding claims, wherein the food authenticity score is indicative of an adulteration status and/or an authenticity status of the analysed food sample.

9. The method of any one of the preceding claims, wherein the authentication score is represented as a probability indicative of the correlation between the food product sample and the one or more food product classes.

10. The method of any one of the preceding claims, wherein the method further comprises the step of generating a scannable QR code comprising information associated with any one, or a combination of: the authentication score, a detected food product class, chemical composition information and nutritional information obtained from the spectral analysis, and adulteration status.

11. The method of claim 10, wherein the method further comprises the step of communicating the QR code to a user via a user interface of a software application running on an electronic device.

12. The method of any one of the preceding claims, wherein the method further comprises the step of at storing authentication information associated with the analysed food product sample in a distributed ledger technology, DLT, running on a cloud computer server, the authentication information comprising any one, or combination, of ; authentication score, food product class associated with the analysed food product sample, an origin information, chemical composition, nutritional information, and location where the food product sample was obtained.

13. The method of any one of the preceding claims, wherein the TIS is generated from a plurality of full scan mass spectrums obtained from a MALDI-TOF-MS spectral analysis of the food product sample.

14. A computer system for evaluating the authenticity of a food product sample, the system comprising a
a user interface running on an electronic device accessible by a user for receiving and providing information;
processing module configured to execute the method of claims 1 to 13 for evaluating the authenticity of a food product sample; and
a communication module configured to output authentication information associated with the analysed food product sample.

15. A computer program product comprising executable instructions, which when executed causes a processing unit to perform the method of claims 1 to 13.
